# EUROPEAN PATENT SPECIFICATION

(11) **EP 3 270 762 B1**
(45) Date of publication and mention of the grant of the patent: **23.01.2019**
(21) Application number: 16719498.4
(22) Date of filing: 14.03.2016
(51) Int. Cl.: A61B 1/267, A61B 1/018

(54) **DEVICE FOR TRACHEAL INTUBATION**
VORRICHTUNG ZUR TRACHEALEN INTUBATION
DISPOSITIF D'INTUBATION TRACHÉALE

(30) Priority: 16.03.2015 IT MO20150060
(43) Date of publication of application: 24.01.2018
(73) Proprietor: Duepuntozero S.r.l., 21100 Varese (IT)
(72) Inventor: FERRARIO, Mario Agostino, 21100 Varese (IT)
(74) Representative: Brunacci, Marco
(86) International application number: PCT/IB2016/051443
(87) International publication number: WO 2016/147107

(56) References cited:
- US-A- 5 163 941
- US-A1- 2010 312 059
- US-A1- 2012 059 223
- US-A1- 2013 014 750
- US-A1- 2013 327 324

## Description

### Technical Field

The present invention relates to a device to perform tracheal intubation.

### Background Art

In the medical field, both within and without hospitals, the need is common to employ devices for tracheal intubation to ensure ventilation in patients with respiratory insufficiency of any origin and/or for the administration of general anesthesia during surgery.

For this purpose various types of devices are known and used, called laryngoscopes, comprising a handle and a blade, which enable an endotracheal tube to be introduced into the patient's trachea, in order to ensure the patency of the airways and ensure the connection of the breathing system with artificial mechanical respirators or manual support systems.

In detail, the laryngoscope is inserted into the patient's mouth, in such a way as to move and lift the tongue, making it possible to directly see the epiglottis and the vocal cords and therefore, to position the endotracheal tube.

To ensure the view and the correct positioning of the endotracheal tube, the devices of known type are equipped with a light source at the extremity of the blade.

To be able to see the vocal cords, other known devices use a camera or an optic fiber at the extremity of the blade and a monitor, which can be external or directly mounted on the handle. The latter almost always provide for the need to insert in the endotracheal tube a semi-rigid and malleable stylet, which impresses on the tube itself a curvature able to reach the vocal cords.

In the devices of known type, the tube and the handle generally represent two separate entities: the operator operates the laryngoscope with one hand and with the other inserts the endotracheal tube.

The patent document US 2011/0270038 describes an instrument that envisages the presence of a groove obtained on the blade, along which a guide element is made to slide for the insertion of the endotracheal tube, and a monitor able to display the images detected by a camera associated with the extremity of the blade itself.

All the devices of known type have a number of drawbacks among which the fact that, for various reasons (among which the shape and dimensions of the blade), they do not allow or make difficult the intubation of patients with a mouth opening of less than 30 mm.

Another drawback of such devices is represented by the fact that the performance of the intubation procedure is made more or less speedy and complex by the anatomical characteristics of the patient and by the mutual position of the patient and the healthcare operator, who is usually positioned behind the head of the patient; this means that in the case of patients who are hard to reach, e.g., victims of traffic accidents blocked inside the vehicle involved in the accident, the procedure is often impossible to perform.

In particular intubation carried out using the known devices often requires the extension or the inclination of the patient's head, a practice contraindicated in the case where patients are involved in accidents with traumas of the spinal column; in fact, in these cases the movement of the head may cause permanent damage to the spinal cord, causing the paralysis and/or the death of the patient. In other cases it is not possible to move the head because of arthrosis phenomena or due to particular anatomic conformations, e.g., serious obesity, which may make intubation extremely difficult or impossible.

As can be easily appreciated, failure to effect intubation associated with the impossibility of ventilation often causes death or significant neurological hypoxic damage.

Furthermore, compared to intubations considered as "difficult", i.e., where the patient has particular anatomic conformations (small mouth, short neck, receding chin ...), malformations, tumors of the tongue, palate, neck, or edema of the soft tissues and/or the vocal cords, or in case of obese patients, the large size of the blade together with its poor manageability makes intubation extremely complex and traumatic for the patient.

In addition, the devices of known type often require the intervention of two healthcare operators, one for the insertion of the laryngoscope and tube and one to take care of passing the necessary instruments to the operator and/or to assist him/her to do the necessary jobs, e.g., to remove the intubation stylet, making the intubation procedure itself more laborious and articulated.

Some devices for tracheal intubation are known from US 2010/312059, US 4,832,020, US 2014/128681, US 2012/059223 and US 5,163,941.

In particular US 2010/312059 describes a device having a handle, which ends with a blade-shaped element intended to be inserted inside a patient's mouth. Both the handle and the blade element have retaining means of the endotracheal tube to be inserted in the patient's mouth. This device is not very easy to use since the blade element and the endotracheal tube associated with it, which have not negligible overall dimensions in relation to the space available, are both inserted simultaneously in the patient's mouth.

US 4,832,020 describes a device for endotracheal intubation which comprises a hollow body, the end of which is intended to be inserted inside the patient's mouth and inside which the endotracheal tube is housed. The endotracheal tube is associated with the body along the inner extension of the same which, even in this case, are therefore inserted simultaneously in the patient's mouth.

US 2014/128681 describes on the contrary a device intended to insert a flexible element inside the patient's mouth. This device has a handle and a blade element, which both have guide means of the flexible element. Once such flexible element is introduced, the entire device shall therefore be removed from the patient's mouth and then the endotracheal tube shall be fitted on the guide element and pushed manually into the patient's trachea.

US 2012/059223 describes a device for endotracheal intubation, which also has a handle, a blade element along which retaining means of the endotracheal tube are defined. The particular feature of this device consists in the presence of means able to vary the curvature of the tube. Even this device has not negligible overall dimensions and thus requires the simultaneous insertion of the device and of the tube inside the patient's mouth.

US 5,163,941 on the contrary describes a device for intubation without the blade element, where the handle has guide means of the endotracheal tube, which is coupled to a semi-rigid and flexible element, used to modify the curvature of the tube and having a light source to recognize its positioning by means of the transillumination of the neck. US 2013/327324 A1 and US 2013/014750 A1 disclose intubation devices according to the preamble of claim 1.

### Description of the Invention

The main aim of the present invention is to provide a device for tracheal intubation that makes the intubation procedure quick, safe, easy and easily performed even in particularly difficult, emergency conditions or where the patient is in a hard-to-reach position.

Within this aim, one object of the present invention is to provide a device for tracheal intubation which makes it possible for a single healthcare operator to perform the intubation procedure, in whatever position the operator is with respect to the patient and in any position of the patient him/herself.

Another object of the present invention is to provide a device for tracheal intubation which allows intubating patients with complex clinical conditions, e.g., who are unable to move their necks, with traumatic injuries, obese, or with a very small mouth opening (even less than 10 mm).

Yet another object of the present invention is to provide a device for tracheal intubation which has several integrated features, i.e., which allows aspirating any biological secretions, administering local anesthetics and at the same time intubating the patient without the aid of additional instruments.

Another object of the present invention is to provide a device which allows to overcome the mentioned drawbacks of the prior art within the ambit of a simple, rational, easy, effective to use and affordable solution.

The above mentioned objects are achieved by the present device for tracheal intubation having the characteristics of claim 1.

### Brief Description of the Drawings

Other characteristics and advantages of the present invention will become better evident from the description of a preferred, but not exclusive, embodiment of a device for tracheal intubation, illustrated by way of an indicative, but nonlimiting, example in the accompanying drawings, wherein:

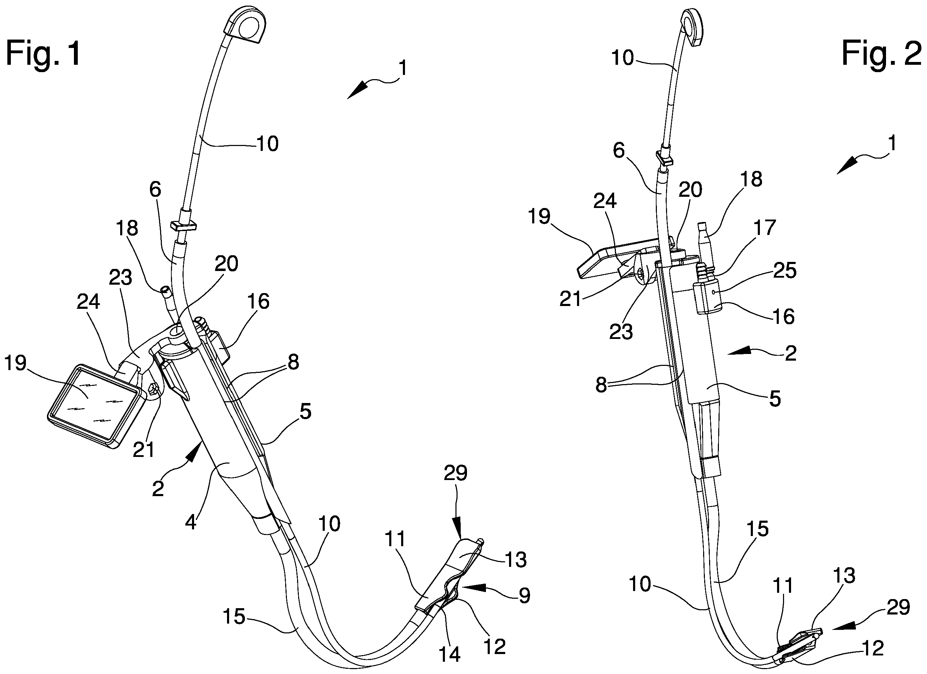

Figure 1 is an axonometric view of a device according to the invention;
Figure 2 is an axonometric view, from another angle, of the device according to the invention;

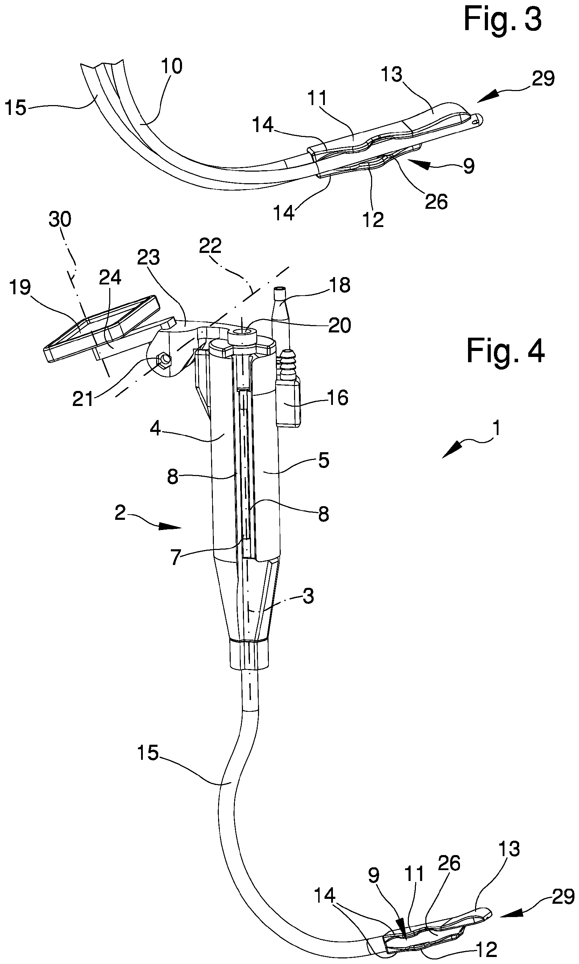

Figure 3 is an axonometric view on an enlarged scale of a detail of Figure 2;
Figure 4 is a front view of the device according to the invention in a first operating configuration;
Figure 5 is a front view of the device according to the invention in a second operating configuration;
Figure 6 is an enlargement of the guide means of the device according to the invention, in a particular embodiment.

### Embodiments of the Invention

The device 1 comprises a handle 2, at least a blade element 29, defining an extremity of the device itself and intended to be introduced inside a patient's mouth, and at least a connecting element 15 interposed between the handle 2 and the blade element 29.

With reference to the particular embodiment shown in the illustrations, the handle 2 has an elongated shape.

Advantageously, the handle 2 has a substantially circular cross section and is symmetrical with respect to a longitudinal axis 3.

Alternative embodiments cannot however be ruled out wherein the handle 2 is asymmetrical with respect to the longitudinal axis 3 and has an ergonomic shape able to make the handle easier and more functional by the healthcare operator. Preferably, the connecting element 15 has a curvilinear extension.

More particularly, the connecting element 15 has a substantially circular section and smaller overall dimensions with respect to the handle 2.

It is specified that in the present treatise expressions such as "top", "bottom", "upper", "lower", "above", "below" and the like, are to be considered with reference to an operating configuration wherein the device 1 has the longitudinal axis 3 arranged vertically.

The device 1 then comprises at least an endotracheal tube 6 intended to be inserted into the patient's trachea. The handle 2 has removable retaining means 4, 5 for retaining a stretch of the endotracheal tube 6.

Preferably, the retaining means 4, 5 are able to retain the proximal extremity of the endotracheal tube 6, i.e., the one closest to the blade element 29. In particular, the retaining means 4, 5 are able to retain the extremity of the endotracheal tube 6 intended to reach the patient's trachea.

Preferably, the retaining means 4, 5 comprise a first portion 4 and a second portion 5 of the handle 2 associated movable with one another so as to define a housing 7 of the endotracheal tube 6.

In particular, as is visible in the illustrations, each portion 4, 5 defines a corresponding wall 8 delimiting the housing 7.

In the embodiment shown in the illustrations, the housing 7 extends substantially parallel to the longitudinal axis 3 of the handle 2. Advantageously, the retaining means 4, 5 comprise elastic means able to counteract the corresponding displacement of the portions 4, 5 in a direction of reciprocal moving away of the corresponding walls 8.

The elastic means, not shown in the illustrations, are of the type of a spring element interposed between the first portion 4 and the second portion 5. Specifically, the second portion 5 is associated movable in rotation with the first portion 4.

More in detail, the second portion 5 is movable in rotation with respect to the first portion 4 in a first direction, in contrast with the elastic means, to allow the insertion of the endotracheal tube 6 in the housing 7, and in a second direction, opposite to the first direction, to allow the retention of the endotracheal tube 6, inserted in the housing 7, due to the force applied by the elastic means.

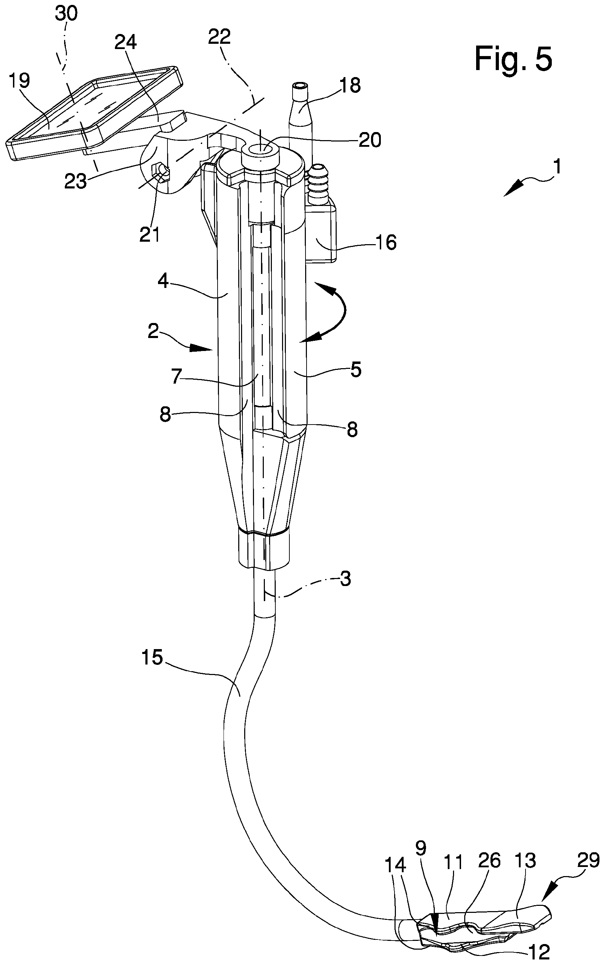

In other words, the first direction of rotation coincides with the direction of mutual moving away of the corresponding walls 8 and is able to allow the widening of the housing 7 to facilitate the insertion of the endotracheal tube 6, while the second direction of rotation coincides with the direction of mutual approach of the walls 8, to perform the retention of the endotracheal tube 6; in particular, the aforementioned mutual moving away and approach directions are shown in Figure 5 by a double arrow. According to the invention, the device 1 comprises a guide element 10 of the flexible type, which is insertable inside the endotracheal tube 6. In particular, the guide element 10 is of the elastically flexible type, which allows it to be easily introduced inside the patient's mouth and to follow its conformation. In this regard it is well to specify that the guide element 10 has a substantially tubular shape.

Furthermore, the device 1 comprises guide means 9 for sliding the guide element 10, which are able to cooperate with a stretch of the guide element itself and are defined on the blade element 29.

According to the invention, the endotracheal tube 6 is released from the guide means 9 and the guide element 10 is released from the connecting element 15.

The blade element 29 is so defined inasmuch as it has two external surfaces 11 and 12, substantially (inasmuch as they can have depressions or irregularities) parallel to one another and such as to define a substantially plate shape which facilitates insertion into the patient's mouth.

More particularly, the blade element 29 has an upper surface 11 and a lower surface 12, each of which has at least a substantially flat corresponding portion. As shown in the illustrations, the upper surface 11 has an extension 13 with respect to the lower surface 12.

In particular, the aforesaid extension 13 is shaped so as to allow the lifting of the tongue and/or the epiglottis of the patient; this is due to the fact that the extension 13 has a thickening with respect to the flat portion of the upper surface itself.

Furthermore, the upper surface 11 and the lower surface 12 have a substantially sinusoidal lateral profile.

As is visible in the illustrations, the guide means 9 define a housing seat 26 of the guide element 10.

With reference to the particular embodiment shown in the illustrations, the housing seat 26 has an elongated shape and is open on one side, to allow the removal of the guide element itself.

Advantageously, the blade element 29 has a detachment wall 14 of the guide element 10.

More in detail, as shown in the illustrations, the detachment wall 14 is defined by two protrusions inclined with respect to the longitudinal extension of the housing seat 26 and diverging towards the outside proceeding towards the extremity of the device 1 defined by the blade element 29.

In the present case, the detachment wall 14 is intended to be contacted by the endotracheal tube 6 as a result of its being released by the retaining means 4, 5 and of its sliding along the guide element 10; this means that the endotracheal tube 6, together with the guide element 10 housed inside it, is pushed away from the housing seat 26.

As mentioned above, the guide element 10 is released from the connecting element 15; this allows the free movement and orientation of the guide element 10. The housing seat 26 therefore extends only along the blade element 29 and is interrupted at the connecting element 15. It is easy to appreciate how the above dimensions together with the freedom of movement, determined by the release of the guide element 10 from the connecting element 15, permit facilitating the movement of the device 1 during use and, therefore, the easy intubation of patients with a reduced mouth opening or with complex clinical situations.

Alternative embodiments cannot however be ruled out wherein the guide means 9 also extend along at least a stretch of the connecting element 15. Furthermore, the device 1 comprises valve means 16 connectable to air aspiration means and to the guide element 10 to allow the aspiration of biological secretions present in the patient's oral cavity.

More particularly, the valve means 16, of the disposable type and known to the expert of the sector, can be associated with the handle 2 in a removable way. The handle 2 therefore has attachment means (not shown in detail in the illustrations) with which the valve means 16 are associated from time to time. Advantageously, the valve means 16 comprise an air flow pipe 17 having a hole 25 accessible from the outside, and able to be placed in communication with the guide element 10, and manually closable by the healthcare operator.

With reference to the particular embodiment shown in the illustrations, the valve means 16 are associated above the handle 2 and comprise a joining element 18 to the aspiration means.

In the present case, the healthcare operator adjusts the flow and direction of the air by resting his/her finger on the hole 25; this means that the operator, by leaving the hole 25 free, allows the air to come out of it, while on the contrary, by pressing his/her finger on the hole 25, he/she determines the aspiration of the biological secretions in the patient's oral cavity. Alternative embodiments cannot however be ruled out wherein the guide element 10 can be used to administrate local anesthesia and/or drugs to the patient.

According to the invention, the device 1 comprises an image display device 19 for displaying images, operatively connectable to image acquisition means 27, not shown in the illustrations, e.g., of the type of a camera or optical fibers. The acquisition means 27 can be associated with at least one of the guide body 9 and the guide element 10. More in detail, the acquisition means 27 can be arranged at the extremity stretch of the guide body 9, e.g., at the lower part of the extension 13, and/or at the extremity of the guide element 10.

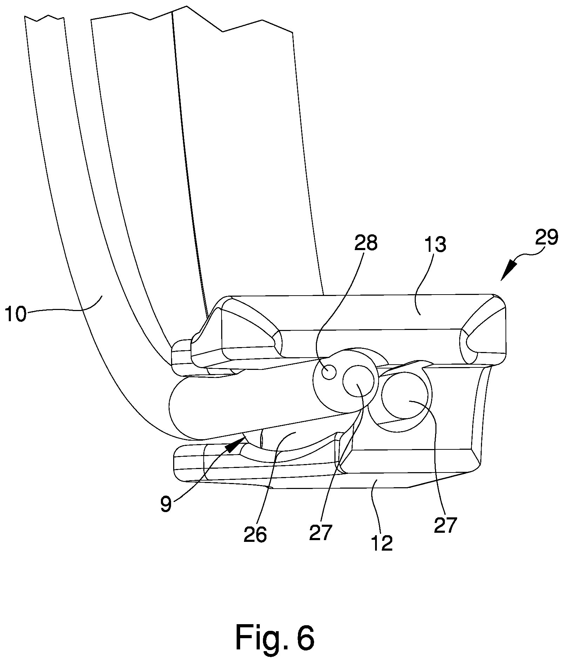

In the case of the acquisition means 27 being associated with both the guide body 9 and the guide element 10, as shown in Figure 6, it is possible to simultaneously obtain both the view of the pharyngeal area and that of the tracheal area.

If the guide element 10 has, besides the acquisition means 27, also an operating channel 28, a traditional bronchoscopy can also be performed with the device 1. With reference to the particular embodiment shown in the illustrations, the display device 19 is a LED screen.

Advantageously, the display device 19 is associated with the handle 2 by interposition of movement means 20, 21 able to move the display device 19 with respect to the handle itself.

The movement means 20, 21 comprise first movement means 20 for the rotation of the display device 19 around a first axis of rotation.

With reference to the particular embodiment shown in the illustrations, the first axis of rotation coincides with the longitudinal axis 3; this means that the aforesaid first movement means 20 are rotatable, by an angle substantially equal to 360°, around the longitudinal axis 3.

Moreover, the movement means 20, 21 comprise second movement means 21 for the rotation of the display device 19 around a second axis of rotation 22, different to the first axis of rotation 3.

Preferably, the second axis 22 is transverse to the first axis 3 and, more particularly, it is perpendicular to the first axis itself.

Advantageously, the second axis 22 is substantially horizontal and allows the movement of the display device 19 upwards or downwards.

In the embodiment shown in the illustrations, the movement means 20, 21 comprise a first support element 23 and a second support element 24.

In detail, the first support element 23 is hinged to the handle 2 around the first axis 3 and the second support element 24 is associated with the LED screen 19; advantageously, the second support element 24 is hinged to the first support element 23 around the second axis 22.

In the preferred embodiment shown in the illustrations, the first support element 23 is shaped so as to allow the positioning of the display device 19 at the housing 7, i.e. in front of the endotracheal tube 6, without interfering with the tube itself.

Preferably, the movement means 20, 21 also comprise third movement means, not visible in detail in the illustrations, for the rotation of the display device 19 around a third axis of rotation, different to the first and the second axis 3, 22, and identified in the illustrations by reference number 30.

More in detail, the third movement means are interposed between the second support element 24 and the display device 19, and therefore allow the rotation of the latter around an axis substantially perpendicular to the LED screen.

It is easy to appreciate how the presence of the movement means 20, 21 allows intubating patients in difficult-to-reach positions, inasmuch as the healthcare operator him/herself maintains the view of the patient's oral cavity during the entire intubation procedure, greatly increasing the functional value of the present invention.

The operation of the present invention is as follows.

Once the device 1 has been gripped by means of the handle 2, the healthcare operator widens the walls 8 of the housing seat 26, moving the second portion 5 along the first direction, and inserts the endotracheal tube 6 in the housing seat 26, after which he/she releases the second portion 5 so as to perform the retention of the tube itself.

Subsequently, the guide element 10 is inserted inside the endotracheal tube 6 and pushed up at the housing seat 26.

Depending on the position taken by the patient and on his/her clinical condition, the operator rotates the display device 19 to obtain the view of the patient's oral cavity.

Then, the blade element 29 is inserted into the patient's oral cavity and, at the same time, the healthcare operator displays the inside by means of the display device 19, which can also be moved during intubation according to the needs of the healthcare operator.

More specifically, once the blade element 29 has been inserted into the patient's mouth, his/her vocal cords can be displayed and the healthcare operator pushes the guide element 10 into the trachea, making it slide along the housing seat 26. During this operation, the endotracheal tube 6 remains fast with respect to the handle 2.

Once the guide element 10 has reached the trachea, the operator releases the endotracheal tube 6 by means of the movement of the retaining means 4, 5 along the first direction.

At this point, the healthcare operator makes the endotracheal tube 6 slide manually on the guide element 10 until it contacts the detachment wall 14. Once the detachment wall 14 has been reached, the endotracheal tube 6 is pushed outwards, thereby dragging the guide element 10 outside the housing seat 26.

The healthcare operator slides the endotracheal tube 6 as far as the extremity of the guide element 10; at this point, the healthcare operator him/herself removes the blade element 29 from the patient's mouth and, lastly, takes the guide element 10 off the endotracheal tube, leaving the latter positioned in the patient's trachea.

It has in practice been ascertained how the described invention achieves the intended objects.

The fact is underlined that the particular solution of providing a flexible guide element released from the connecting element allows reducing the diameters of the latter, obtaining a device for tracheal intubation which is compact and reduced in size and, furthermore, which makes the intubation procedure fast, easy and easily performed even in emergency conditions, or in the case of the patient being in difficult-to-reach positions.

To this must be added the fact that the presence of the endotracheal tube retaining means associated with the body enables the intubation procedure to be performed by a single healthcare operator, in whatever position the operator is with respect to the patient him/herself.

The device to which the present invention refers, therefore, permits inserting the guide element in a practical and easy way inside the patient's trachea, minimizing any inconvenience thanks to the particular conformation of the device, and subsequently inserting the endotracheal tube once the guide element has reached the desired position.

## Claims

1. Device (1) for tracheal intubation, comprising:
- at least a handle (2);
- at least a blade element (29) defining an extremity of said device (1) and intended to be introduced inside a patient's mouth;
- at least a connecting element (15) interposed between said handle (2) and said blade element (29);
- at least an endotracheal tube (6) intended to be inserted into a patient's trachea;
- at least a flexible guide element (10) which is insertable inside said endotracheal tube (6);
- guide means (9) for sliding said guide element (10), able to cooperate with at least a stretch of the guide element (10) and defined on said blade element (29), said endotracheal tube (6) being released from said guide means (9) and said guide element (10) being released from said connecting element (15);
- at least an image display device (19) operatively connectable to image acquisition means (27) associable with at least one of said guide means (9) and said guide element (10);
**characterized by** the fact that said handle (2) has retaining means (4, 5) for removably retaining at least a stretch of said endotracheal tube (6), where said retaining means (4, 5) comprise at least a first (4) and a second portion (5) of said handle (2) associated movable with respect to each other so as to define a housing (7) for the endotracheal tube (6).

2. Device (1) according to claim 1, **characterized by** the fact that each of said portions (4, 5) defines a respective wall (8) delimiting said housing (7), and by the fact that said retaining means (4, 5) comprise elastic means able to counteract the displacement of said portions (4, 5) moving in a direction of mutual displacement of the respective walls.

3. Device (1) according to claim 2, **characterized by** the fact that said second portion (5) is associated movable in rotation with respect to said first portion (4) and is movable with respect to it in a first direction, in contrast to said elastic means, to allow the insertion of the endotracheal tube (6) in said housing (7), and in a second direction, opposite to said first direction, to allow the retention of the endotracheal tube (6) inserted in said housing (7) due to the force applied by said elastic means.

4. Device (1) according to one or more of the preceding claims, **characterized by** the fact that said guide means (9) define a housing seat (26) of said guide element (10) having an elongated shape and open on one side to allow the removal of the guide element.

5. Device (1) according to claim 4, **characterized by** the fact that said blade element (29) defines at least a detachment wall (14), intended to be contacted by the endotracheal tube (6) as a result of its sliding along said guide element (10) and arranged inclined with respect to the longitudinal extension of said housing seat (26).

6. Device (1) according to one or more of the preceding claims, **characterized by** the fact that said connecting element (15) has a curvilinear extension.

7. Device (1) according to one or more of the preceding claims, **characterized by** the fact that said connecting element (15) has a substantially circular section and smaller overall dimensions with respect to said handle (2).

8. Device (1) according to one or more of the preceding claims, **characterized by** the fact that said display device (19) is associated with said handle (2) by interposition of movement means (20, 21) able to move said display device (19) with respect to said handle (2).

9. Device (1) according to claim 8, **characterized by** the fact that said movement means (20, 21) comprise first movement means (20) for the rotation of said display device (19) around a first axis of rotation (3).

10. Device (1) according to claim 9, **characterized by** the fact that said movement means (20, 21) comprise second movement means (21) for the rotation of said display device (19) around a second axis of rotation (22) different to said first axis (3).

11. Device (1) according to claim 10, **characterized by** the fact that said movement means (20, 21) comprise third movement means for the rotation of said display device (19) around a third axis of rotation (30) different to said first and second axis (3, 22).

12. Device (1) according to one or more of the preceding claims, **characterized by** the fact that it comprises valve means (16) connectable to aspiration means and to said guide element (10) to allow the aspiration of biological secretions present in the patient's oral cavity.

13. Device (1) according to claim 12, **characterized by** the fact that said valve means (16) are associated with said handle (2) in a removable way and comprise at least an air flow pipe (17) having at least a hole (25) accessible from the outside and manually closable by an operator.

## Patentansprüche

1. Vorrichtung (1) zur trachealen Intubation, umfassend:
- mindestens einen Griff (2);
- mindestens ein Blattelement (29), das ein Ende der Vorrichtung (1) definiert und zum Einführen in den Mund eines Patienten vorgesehen ist;
- mindestens ein Verbindungselement (15), das zwischen dem Griff (2) und dem Blattelement (29) eingefügt ist;
- mindestens einen endotrachealen Schlauch (6), der zum Einführen in die Luftröhre eines Patienten vorgesehen ist;
- mindestens ein flexibles Führungselement (10), das in das Innere des endotrachealen Schlauchs (6) einführbar ist;
- Führungsmittel (9) zum Verschieben des Führungselements (10), geeignet, um mit mindestens einem Abschnitt des Führungselements (10) zusammenzuwirken und definiert an dem Blattelement (29), wobei der endotracheale Schlauch (6) von den Führungsmitteln (9) gelöst wird und das Führungselement (10) von dem Verbindungselement (15) gelöst wird;
- mindestens eine Bildanzeigevorrichtung (19), die wirksam mit Bilderfassungsmitteln (27) verbindbar ist, welche mit mindestens einem der Führungsmittel (9) und dem Führungselement (10) verbindbar sind;
**dadurch gekennzeichnet, dass** der Griff (2) Haltemittel (4, 5) zum lösbaren Halten mindestens eines Abschnitts des endotrachealen Schlauchs (6) aufweist, wobei die Haltemittel (4, 5) mindestens einen ersten (4) und einen zweiten (5) Abschnitt des Griffs (2) umfassen, die beweglich bezüglich einander verbunden sind, um ein Gehäuse (7) für den endotrachealen Schlauch (6) festzulegen.

2. Vorrichtung (1) nach Anspruch 1, **dadurch gekennzeichnet, dass** jeder der Abschnitte (4, 5) jeweils eine Wand (8) definiert, die das Gehäuse (7) begrenzt, und dass die Haltemittel (4, 5) elastische Mittel umfassen, die geeignet sind, um der Verschiebung der Abschnitte (4, 5) entgegenzuwirken, die sich in eine Richtung gegenseitiger Verschiebung der jeweiligen Wände bewegen.

3. Vorrichtung (1) nach Anspruch 2, **dadurch gekennzeichnet, dass** der zweite Abschnitt (5) drehbeweglich in Bezug auf den ersten Abschnitt (4) verbunden ist und in Bezug dazu, im Gegensatz zu den elastischen Mitteln, in einer ersten Richtung beweglich ist, um das Einführen des endotrachealen Schlauchs (6) in das Gehäuse (7) zu ermöglichen, und in einer zweiten Richtung entgegengesetzt zu der ersten Richtung beweglich ist, um das Halten des in das Gehäuse (7) eingeführten endotrachealen Schlauchs (6) durch die von den elastischen Mitteln aufgebrachte Kraft zu ermöglichen.

4. Vorrichtung (1) nach einem oder mehreren der vorhergehenden Ansprüche, **dadurch gekennzeichnet, dass** die Führungsmittel (9) einen Gehäusesitz (26) des Führungselements (10) mit einer länglichen Form und offen an einer Seite definieren, um das Entfernen des Führungselements zu ermöglichen.

5. Vorrichtung (1) nach Anspruch 4, **dadurch gekennzeichnet, dass** das Blattelement (29) mindestens eine Ablösewand (14) festlegt, die dazu vorgesehen ist, von dem endotrachealen Schlauch (6) als Resultat von dessen Verschieben entlang des Führungselements (10) kontaktiert zu werden und in Bezug auf die Längserstreckung des Gehäusesitzes (26) geneigt angeordnet zu sein.

6. Vorrichtung (1) nach einem oder mehreren der vorhergehenden Ansprüche, **dadurch gekennzeichnet, dass** das Verbindungselement (15) eine gekrümmte Erstreckung aufweist.

7. Vorrichtung (1) nach einem oder mehreren der vorhergehenden Ansprüche, **dadurch gekennzeichnet, dass** das Verbindungselement (15) einen im Wesentlichen kreisförmigen Querschnitt und kleinere Gesamtabmessungen in Bezug auf den Griff (2) aufweist.

8. Vorrichtung (1) nach einem oder mehreren der vorhergehenden Ansprüche, **dadurch gekennzeichnet, dass** die Anzeigevorrichtung (19) durch Einfügen von Bewegungsmitteln (20, 21), die geeignet sind, die Anzeigevorrichtung (19) in Bezug auf den Griff (2) zu bewegen, mit dem Griff (2) verbunden ist.

9. Vorrichtung (1) nach Anspruch 8, **dadurch gekennzeichnet, dass** die Bewegungsmittel (20, 21) erste Bewegungsmittel (20) zum Drehen der Anzeigevorrichtung (19) um eine erste Drehachse (3) umfassen.

10. Vorrichtung (1) nach Anspruch 9, **dadurch gekennzeichnet, dass** die Bewegungsmittel (20, 21) zweite Bewegungsmittel (21) zum Drehen der Anzeigevorrichtung (19) um eine von der ersten Achse (3) verschiedene zweite Drehachse (22) umfassen.

11. Vorrichtung (1) nach Anspruch 10, **dadurch gekennzeichnet, dass** die Bewegungsmittel (20, 21) dritte Bewegungsmittel zum Drehen der Anzeigevorrichtung (19) um eine von der ersten und zweiten Achse (3, 22) verschiedene dritte Drehachse (30) umfassen.

12. Vorrichtung (1) nach einem oder mehreren der vorhergehenden Ansprüche, **dadurch gekennzeichnet, dass** sie Ventilmittel (16) umfasst, die mit Aspirationsmitteln und dem Führungselement (10) verbindbar sind, um die Aspiration von in der Mundhöhle des Patienten vorhandenen biologischen Sekreten zu ermöglichen.

13. Vorrichtung (1) nach Anspruch 12, **dadurch gekennzeichnet, dass** die Ventilmittel (16) lösbar mit dem Griff (2) verbunden sind und mindestens einen Luftstromschlauch (17) mit mindestens einer Öffnung (25) umfassen, die von außen zugänglich und von einem Bediener von Hand schließbar ist.

## Revendications

1. Dispositif (1) d'intubation trachéale, comprenant :
au moins un manche (2) ;
au moins un élément de pale (29) définissant une extrémité dudit dispositif (1) et destiné à être introduit à l'intérieur de la bouche d'un patient ;
au moins un élément de raccord (15) interposé entre ledit manche (2) et ledit élément de pale (29) ;
au moins un tube endotrachéal (6) destiné à être inséré dans la trachée d'un patient ;
au moins un élément de guidage souple (10) qui peut être inséré à l'intérieur dudit tube endotrachéal (6) ;
des moyens de guidage (9) pour faire glisser ledit élément de guidage (10), pouvant coopérer avec au moins un bout de l'élément de guidage (10) et défini sur ledit élément de raccord (29), le tube endotrachéal (6) étant relâché depuis lesdits moyens de guidage (9) et ledit élément de guidage (10) étant relâché depuis ledit élément de raccord (15) ;
au moins un dispositif d'affichage d'image (19) pouvant être raccordé de manière opérationnelle à des moyens d'acquisition d'image (27) pouvant être associés avec au moins l'un parmi lesdits moyens de guidage (9) et ledit élément de guidage (10) ;
**caractérisé en ce que** ledit manche (2) possède des moyens de retenue (4, 5) pour retenir de manière amovible au moins un bout dudit tube endotrachéal (6), où lesdits moyens de retenue (4, 5) comprennent au moins une première (4) et une deuxième (5) partie dudit manche (2) associé de manière mobile l'une par rapport à l'autre de sorte à définir un logement (7) pour le tube endotrachéal (6).

2. Dispositif (1) selon la revendication 1, **caractérisé en ce que** chacune desdites parties (4, 5) définit une paroi respective (8) délimitant ledit logement (7), et
**en ce que** lesdits moyens de retenue (4, 5) comprennent des moyens élastiques pouvant empêcher le déplacement desdites parties (4, 5) en mouvement dans une direction de déplacement mutuel des parois respectives.

3. Dispositif (1) selon la revendication 2, **caractérisé en ce que** ladite deuxième partie (5) est associée de manière mobile en rotation par rapport à ladite première partie (4) et est mobile par rapport à celle-ci dans une première direction, à l'inverse des moyens élastiques, pour permettre l'insertion du tube endotrachéal (6) dans ledit logement (7), et dans une deuxième direction, opposée à ladite première direction, pour permettre la rétention du tube endotrachéal (6) inséré dans ledit logement (7) en raison de la force appliquée par lesdits moyens élastiques.

4. Dispositif (1) selon l'une ou plusieurs des revendications précédentes, **caractérisé en ce que** lesdits moyens de guidage (9) définissent un siège de logement (26) dudit élément de guidage (10) possédant une forme allongée et ouvert sur un côté pour permettre le détachement de l'élément de guidage.

5. Dispositif (1) selon la revendication 4, **caractérisé en ce que** ledit élément de pale (29) définit au moins une paroi de détachement (14), destinée à être mise en contact avec le tube endotrachéal (6) du fait de son glissement le long dudit élément de guidage (10) et disposée de manière inclinée par rapport à l'extension longitudinale dudit siège de logement (26).

6. Dispositif (1) selon l'une ou plusieurs des revendications précédentes, **caractérisé en ce que** ledit élément de raccord (15) possède une extension curvilinéaire.

7. Dispositif (1) selon l'une ou plusieurs des revendications précédentes, **caractérisé en ce que** ledit élément de raccord (15) possède une section sensiblement circulaire et des dimensions générales réduites par rapport audit manche (2).

8. Dispositif (1) selon l'une ou plusieurs des revendications précédentes, **caractérisé en ce que** ledit dispositif d'affichage (19) est associé audit manche (2) par l'interposition de moyens de mouvement (20, 21) pouvant déplacer ledit dispositif d'affichage (19) par rapport audit manche (2).

9. Dispositif (1) selon la revendication 8, **caractérisé en ce que** lesdits moyens de mouvement (20, 21) comprennent des premiers moyens de mouvement (20) pour la rotation dudit dispositif d'affichage (19) autour d'un premier axe de rotation (3).

10. Dispositif (1) selon la revendication 9, **caractérisé en ce que** lesdits moyens de mouvement (20, 21) comprennent des deuxièmes moyens de mouvement (21) pour la rotation dudit dispositif d'affichage (19) autour d'un deuxième axe de rotation (22) différent du premier axe (3).

11. Dispositif (1) selon la revendication 10, **caractérisé en ce que** lesdits moyens de mouvement (20, 21) comprennent des troisièmes moyens de mouvement pour la rotation du dispositif d'affichage (19) autour d'un troisième axe de rotation (30) différent desdits premier et deuxième axes (3, 22).

12. Dispositif (1) selon l'une ou plusieurs des revendications précédentes, **caractérisé en ce qu'**il comprend des moyens de valve (16) pouvant être raccordés à des moyens d'aspiration et audit élément de guidage (10) pour permettre l'aspiration de sécrétions biologiques présentes dans la cavité orale du patient.

13. Dispositif (1) selon la revendication 12, **caractérisé en ce que** lesdits moyens de valve (16) sont associés audit manche (2) de manière détachable et comprennent au moins un tuyau d'écoulement d'air (17) possédant au moins un trou (25) accessible de l'extérieur et pouvant être fermé manuellement par un opérateur.
